# EUROPEAN PATENT SPECIFICATION

(11) **EP 1 706 167 B1**
(45) Date of publication and mention of the grant of the patent: **09.05.2018**
(21) Application number: 05711616.2
(22) Date of filing: 21.01.2005
(51) Int. Cl.: A61M 25/10, A61M 25/00

(54) **CUFF RESISTANT FOLEY CATHETER**
FOLEY-KATHETER OHNE MANSCHETTENBILDUNG
SONDE DE FOLEY RESISTANT A L'ENGORGEMENT

(30) Priority: 22.01.2004 US 539054 P; 20.01.2005 US 39074
(43) Date of publication of application: 04.10.2006
(73) Proprietor: Rochester Medical Corporation, Stewartville, MN 55976 (US)
(72) Inventor: CONWAY, Anthony, J., Chatfield, MN 55923 (US)
(74) Representative: Pawlyn, Anthony Neil
(86) International application number: PCT/US2005/001606
(87) International publication number: WO 2005/072808

(56) References cited:
- EP-A- 0 553 960
- WO-A-95/09667
- US-A- 5 409 495
- US-A- 5 599 321
- US-A1- 2002 032 406

## Description

### FIELD OF THE INVENTION

The invention relates to a Foley-type catheter constructed to reduce or eliminate the retention balloon from cuffing. More particularly, in certain embodiments, the invention relates to a catheter Including a sheath layer over the outside surface of the catheter, and to methods of making and using such a catheter. In an embodiment, the invention relates to a catheter with a retention balloon including ribs.

### BACKGROUND OF THE INVENTION

Foley-type catheters are tube like devices that are used to drain urine from a patient's bladder. Foley catheters are inserted through the urethra and typically held in place with an Inflatable balloon. The balloon is in a deflated position when the catheter is first inserted. Then, once the catheter is in the proper position, the balloon is inflated with a fluid. The inflated balloon is larger than the diameter of the urethra and thereby physically prevents the catheter from being removed. Foley catheters are also known as "indwelling" catheters because they are designed to be left in place for a period of time.

Latex rubber is commonly used for Foley catheters. However, latex rubber can be problematic as many patients have latex allergies. Silicone rubber has been used to make Foley catheters since it does not cause the same problems with irritation as does latex rubber. However, silicone rubber does not have the same elastic properties as latex rubber. As a result, the balloons on Foley catheters that are made with silicone rubber can exhibit "cuffing."

Cuffing refers to the situation in which the balloon tends to be shifted toward the bladder end of the catheter as the balloon itself is pressed against the bladder wall when holding the catheter in place. Since the balloon is attached at its end to the shaft of the catheter, the balloon can form a cuff as the outer expanded portion of the balloon is pushed over the inner attached end of the balloon. This cuff can remain when the balloon is deflated before withdrawal of the catheter from the patient. The cuff results in the deflated balloon having a larger diameter than it did when it was first inserted. The increased diameter can result in discomfort and injury to patients. Accordingly, a need exists for a silicone rubber Foley catheter that resists cuffing.

US5599321 provides a catheter according to the pre-characterizing part of claim 1. WO95/09667, USS409495 and US2002/0032406 provide examples of other catheter designs.

### SUMMARY OF THE INVENTION

The invention relates to a Foley-type catheter constructed to reduce or eliminate retention balloon cuffing. More particularly, in certain embodiments, the invention relates to a catheter including a sheath layer over the outside surface of the catheter and to methods of making such a catheter.

According to a first aspect the invention provides a catheter comprising:
a central layer providing a catheter shaft and having a catheter tip, an exterior surface and an interior surface and defining a first lumen and a second lumen;
a balloon layer surrounding the central layer, the balloon layer including a balloon portion, the balloon portion being the portion which is not bonded to the exterior surface of the central layer; the balloon portion being in the form of a cavity disposed between the exterior surface of the central layer and the balloon layer;
wherein the balloon cavity is in fluid communication with the second lumen;
wherein the balloon layer and the central layer are joined together at a distal end and at a proximal end of the balloon cavity relative to the catheter tip; and
a sheath layer surrounding the balloon layer and extending at least over the balloon layer;
   characterized in that, the balloon layer Is provided on the catheter shaft just in the area of the balloon portion and the sheath layer is provided on the catheter shaft over the entire length of the catheter shaft and the balloon layer does not cover the entire length of the catheter shaft to be inserted into the patient.

In an embodiment, the invention may further provide a catheter with a retention balloon including ribs.

### BRIEF DESCRIPTION OF THE FIGURES

In the drawings, in which like reference numerals indicate corresponding parts throughout the several views,
FIG. 1A is a schematic view of a catheter is an original deflated configuration;
FIG. 1B is a schematic view of a catheter in an inflated position wherein the balloon is cuffing;
FIG. 1C is a schematic view of a catheter in a deflated position wherein the balloon has retained a cuff;
FIG. 2 is a partial cut-away view of a portion of a Foley catheter made in accordance with an embodiment of the present invention;
FIG. 3 shows a cross-sectional view of an embodiment of the invention formed with ribs made of a compound different from the balloon itself;
FIG. 4 is a partial cut-away view of an extruded double lumen tube in partial cross-section;
FIG. 5 is a cross-sectional view of the extruded double lumen tube as seen from the line 202-202' of FIG. 4;
FIG. 6 is a partial cut-away view of the tube shown in FIG. 4 after an opening is punched in the outer surface;
FIG. 7 is a cross-sectional view of the tube as shown from the line 204-204' of FIG. 6;
FIG. 8 is a partial cut-away view of the double lumen tube shown in FIG. 6 after a portion of the first lumen has been filled with a polymeric bonding composition;
FIG. 9 is a cross-sectional view of the tube as seen from the line 206-206' of FIG. 8;
FIG. 10 is a partial cut-away view of the double lumen tube shown in FIG. 8 after a tip is affixed to a distal end of the tube;
FIG. 11 is a schematic view of a portion of a rack used to retain a plurality of tubes during a series of steps designed to provide the tube with an overcoat layer of a polymeric bonding composition;
FIG. 12 is a partial cut-away view of an intermediate tube similar to the tube shown in FIG. 10 at an intermediate stage of manufacture prior to the first of a series of dipping steps;
FIG. 13 is a partial cut-away view of an intermediate tube similar to that shown in FIG. 12, but following a first dipping step wherein the outer surface is coated with a bond preventing agent up to the point designated by line A;
FIG. 14 is a cross-sectional view of the intermediate tube of FIG. 13 as shown from the line 211-211';
FIG. 15 is a partial cut-away view of an intermediate tube similar to that shown in FIG. 13, but after a subsequent dipping step or steps in which the coating of bond preventing agent on a portion of the outer surface of the intermediate tube has been removed;
FIG. 16 is a partial cut-away view of an intermediate tube similar to that shown in FIG. 15, but after subsequent steps in which a balloon compound has been deposited both slightly above and below the bond preventing agent;
FIG. 17 is a partial cut-away view of an intermediate tube similar to that shown in FIG. 16, but after a step of dipping the entire length of the catheter shaft in a balloon compound;
FIG. 18 is a partial cut-away view of a Foley catheter made in accordance with the present invention following testing and cleaning and showing cut-away views of portions thereof;
FIG. 19 is a partial cut-away view of a portion of the Foley catheter shown in FIG. 18, but with the balloon portion of the catheter shown when expanded;
FIG. 20 is a cross-sectional view of a Foley catheter made in accordance with the present invention showing ribs formed in the balloon portion of the catheter;
FIG. 21 is a partial cut-away view of a portion of an embodiment of a Foley catheter with a finish layer.
FIG. 22 is a schematic illustration of an apparatus used to automate the production of balloon catheters in accordance with the present invention.

While the invention is susceptible to various modifications and alternative forms, specifics thereof have been shown by way of example and drawings, and will be described in detail. It should be understood, however, that the invention is not limited to the particular embodiments described. On the contrary, the intention is to cover modifications, equivalents, and alternatives falling within the spirit and scope of the invention.

### Detailed Description of the Invention

### Cuffing

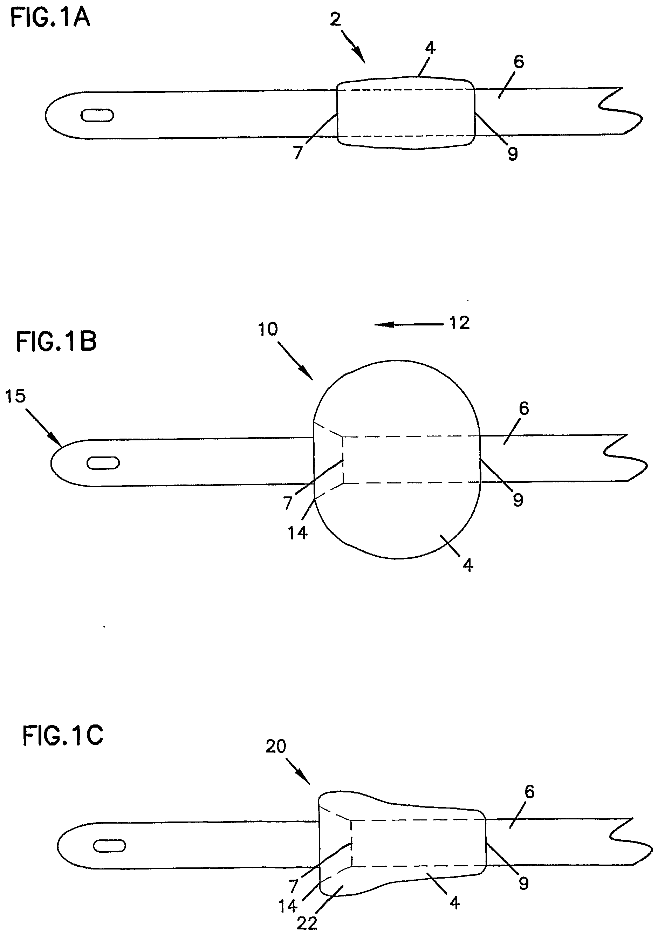

As described above, balloon catheters made with silicone rubber can exhibit problematic cuffing. FIG. 1A shows a schematic view of a catheter in a deflated configuration 2 and illustrates how the balloon 4 is next to the catheter shaft 6. In this configuration, the balloon 4 does not overlap either its distal end 7 or its proximal end 9. Further, in the configuration shown in FIG. 1A, the balloon 4 adds only a small increment to the diameter of the catheter shaft 6 because of how the uninflated balloon 4 lies flat over the catheter shaft 6.

However, as described above, balloon catheters made with silicone rubber may exhibit problems with cuffing. FIG. 1B is a schematic view of a catheter in an inflated position 10 wherein the balloon 4 is cuffing. Cuffing refers to the situation in which the balloon 4 tends to be shifted toward the bladder end 15 of the catheter (in the direction of arrow 12) forming a cuff 14, as the balloon 4 itself is pressed against the bladder wall when holding the catheter in place. Since the balloon 4 is attached at its distal end 7 to the shaft of the catheter 6, the balloon forms a cuff 14 as the outer expanded portion of the balloon 4 is pushed over the inner attached distal end 7 of the balloon 4.

The cuff 14 that is formed tends to remain when the balloon 4 is deflated before withdrawal of the catheter from the patient. FIG. 1C is a schematic view of a catheter in a deflated position 20 after having been inflated wherein the balloon has a cuff 14. The cuff 14 results in the deflated balloon 4 having a larger diameter in an area 22 of the balloon 4 over the cuff 14 than it did when it was first inserted. For example, a balloon that has cuffed may be 12 French sizes larger at the cuff than the actual catheter shaft. The increased diameter can result in discomfort and injury to patients.

### Cuff Resistant Catheters

The present inventors have created embodiments of catheters that can resist cuffing. In an embodiment of the invention, an inner layer is formed over the shaft just in the area of the balloon. Then an outer layer is formed over the entire length of the shaft by dipping it in silicone balloon compound. In this manner, the difference between the diameter of the balloon area and the diameter of the catheter shaft in the finished product can be controlled simply by adjusting the thickness of the inner layer. This is because in contrast to the inner layer, the outer layer covers the entire length of the shaft. Therefore, the balloon area can be thickened by adding to the outer layer while not affecting the relative difference in diameters between the balloon area and the catheter shaft. While not intending to be bound by theory, it is believed that the added thickness in the balloon area results in a silicone rubber balloon that resists cuffing.

In certain embodiments, the effective diameter of the shaft area and the diameter of the balloon area are increased commensurately so that the differential between the shaft diameter and balloon diameter can be kept to an advantageous small amount, for example, about 4 French sizes (e.g., 1.33 mm / 0.052 inch) or less than or equal to 4 French sizes (1.33 mm / 0.052 inch).

The approach of covering the entire catheter shaft with balloon compound can also result in a softer and therefore more comfortable shaft as a balloon compound can be used for the outer layer that has a durometer rating of approximately 20 when cured in contrast to the double lumen tube itself which has a durometer rating of approximately 65. Moreover, this approach can result in a stronger balloon that is less likely to burst.

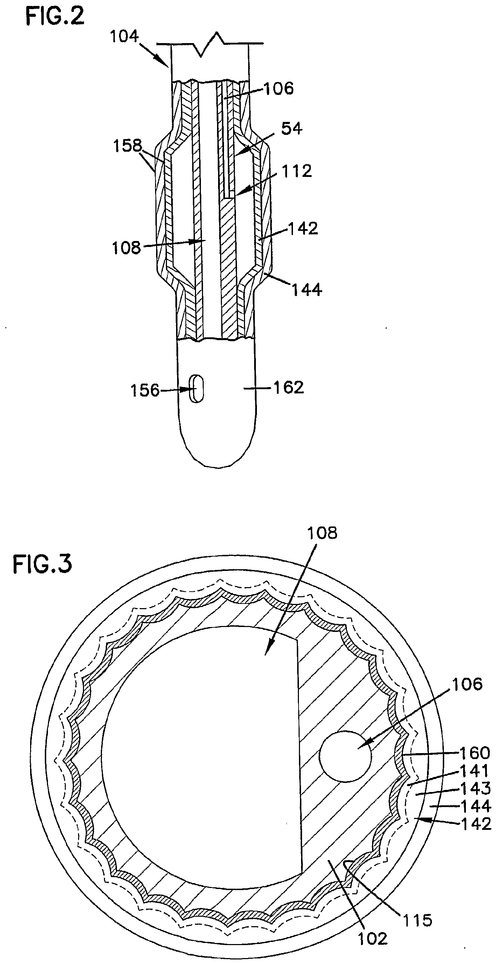

Referring to FIG. 2, a partial cut-away view of a portion of a Foley catheter is shown in accordance with an embodiment of the present invention. The catheter has a finished balloon catheter shaft 104 and a fluid conduit access opening 156 in an exterior surface 162 of the catheter shaft 104. The balloon portion 158 includes sections of two layers including the balloon layer 142 and the sheath layer 144. The balloon cavity 154 is disposed under the balloon layer 142 and is in fluid communication with a capillary lumen 106 via a capillary lumen access opening 112. The Foley catheter also includes a fluid conduit lumen 108.

In an embodiment, the balloon layer is an integral part of the catheter. For example, a balloon layer that is an integral part of the catheter can be formed by the dipping and stripping methods described herein. For example, a balloon layer that is formed from cured material that is applied in cured form to a mandrel or shaft (e.g., as a preformed sleeve or as a tape wound around the shaft) is not an integral part of the catheter.

In an embodiment of the invention, the balloon is formed with ribs made of a compound different from the balloon itself. The compound of the ribs stretches less easily than the compound of the balloon and results in a balloon that resists cuffing. In an embodiment, the ribs fit in corresponding grooves in the main shaft of the catheter so that when the balloon is deflated it does not add to the outside diameter of the balloon.

FIG. 3 shows a cross-sectional view of an embodiment of the invention formed with ribs made of a compound different from the balloon itself. A double lumen tube 102 includes a capillary lumen 106 and a fluid conduit lumen 108. The double lumen tube 102 has undulations or channels 115 on its exterior surface. A balloon layer 142, surrounding the double lumen tube 102, includes a first region 141 and a second region 143. The first region 141 includes ribs 160 including a less pliable silicone rubber than the silicone rubber of the second region 143. When the balloon is in a deflated position, the ribs 160 can fit in the undulations or channels 115.

### Methods of Making

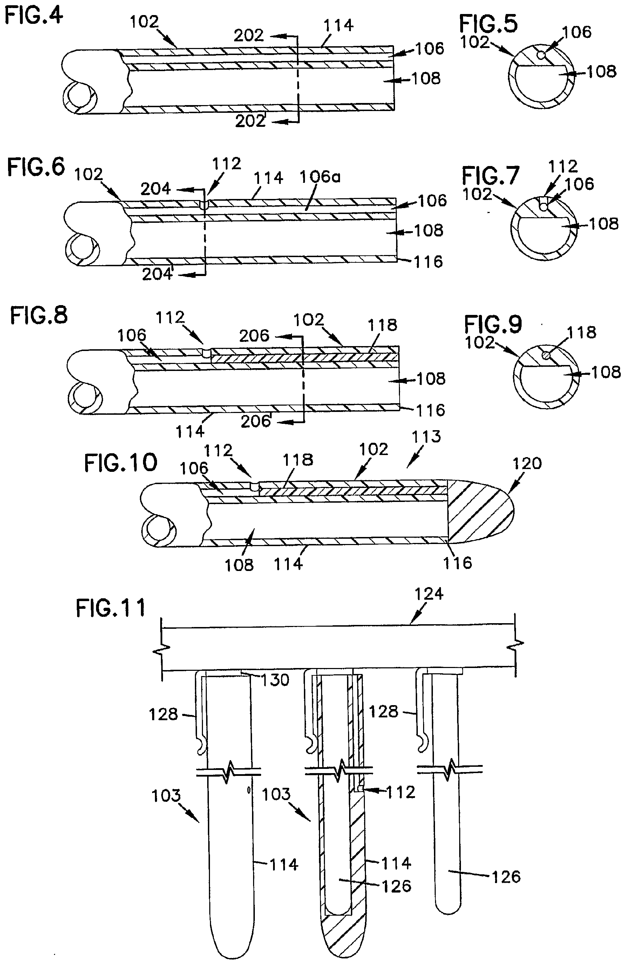

Referring now to the drawings, and specifically to FIGS. 4 and 5, the first step in making a balloon catheter in accordance with the present invention is providing a double lumen tube 102, which is typically extruded. However, the double lumen tube can be made by any known process which yields a double lumen tube. The double lumen tube 102 includes a smaller capillary lumen 106 and a larger fluid conduit lumen 108. The tube includes a resilient polymeric material. In an embodiment, the polymeric material is a biocompatible polymeric material that can be inserted into a human body cavity. In a particular embodiment, the tube includes silicone rubber.

Referring now also to FIGS. 6 and 7, after the double lumen tube is cut to a desired size, a capillary lumen access opening 112 is created in an outer surface 114 of the double lumen tube 102. The capillary lumen access opening 112 communicates with the capillary lumen 106.

Referring now to FIGS. 8-10, an intermediate tube 103 is subsequently prepared from the double lumen tube 102 shown in FIG. 6. In the first step of this process, a measured amount of a polymeric bonding composition, such as silicone rubber or another suitable polymeric bonding material, is injected into the capillary lumen 106 from the distal end 116 of the double lumen tube 102, so that the capillary lumen 106 is filled with a polymeric fill material 118 up to a point just below the capillary lumen access opening 112. A tip 120, such as a rounded silicone rubber tip, can then be affixed to the distal end 116 of the tube 102 to complete the formation of the intermediate tube 103 shown in FIG. 10. In a method of the invention, the distal end 116 of the tube 102 is inserted into a molding apparatus (not shown) designed to mold a tip 120 on the end of the tube 102.

Referring now also to FIGS. 10-17, an embodiment of the process of the invention involves securing a plurality of intermediate tubes 103, like the intermediate 103 shown in FIG. 10, to a rack or pallet 124 as shown in FIG. 11. The rack or pallet 124 will include a plurality of support rods 126, each equipped with a retaining clip 128. The intermediate tubes 103 are secured on the support rods 126 by engaging individual support rods 126 in the larger of the two lumens, called the fluid conduit lumen 108, and sliding the intermediate tubes 103 up over the support rods 126 until the proximal ends 130 of the intermediate tubes 103 abut against the base of the retaining clips 128 or the tip 120 of each of the intermediate tubes 103 fits snugly against the distal tip of each of the support rods 126. Although not shown, it is believed that the intermediate tubes 103 can be secured on the support rods 126 without the aid of the retaining clips 128. This is because extruded double lumen tubes 102 used to make the intermediate tubes 103 generally have a slight bend in one direction or another when they are hung. This results in a slight bend in the intermediate tubes 103 that permits the intermediate tube 103 to be secured on a support rod 126 without the aid of a clip 128.

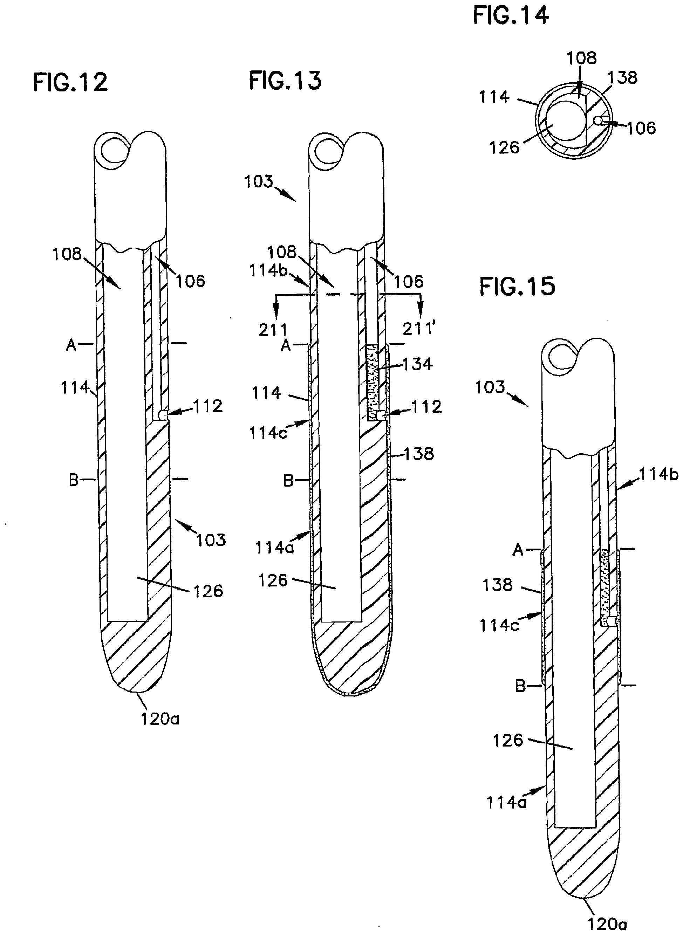

When the intermediate tubes 103 have been secured on the support rods 126, the pallet 124 can be transferred from place to place, and the intermediate tubes 103 on the pallet 124 can be dipped in a series of baths prepared to accomplish a series of process steps. In an embodiment of the method of the invention, the intermediate tube 103 is made entirely of silicone rubber and is secured upon a support rod 126 made of spring steel. The tip 120 and the fill material 118 of the intermediate tube 103 shown in FIG. 10 can be of the same material (silicone rubber) as the double lumen tube 102. Therefore, the tip 120 and the fill material 118 can form integral portions of the intermediate tube 103, which is shown in FIGS. 12-17 as an integral polymeric unit made of a single material.

The first step in the automated coating or dipping process of forming the balloon portion of the balloon catheter, after the intermediate tubes 103 are secured to the pallet 124, is to coat the intermediate tubes 103 with a bond preventing agent, such as a removable bond preventing agent. In an embodiment, this is accomplished by dipping each of the tubes 103 on the pallet 124 simultaneously into a first dip tank containing a bath of a removable bond preventing agent, such as a material which forms a semi-solid film on surfaces when cooled on contact followed by an opportunity for drying. Examples of such materials include petroleum jelly or petrolatum, other oil base substances which form a semisolid upon cooling to room temperature, liquid soaps which dry to form a semi-solid, aqueous soap or detergent solutions, aqueous or oil based film forming solids emulsions, and the like. In one embodiment described herein, hot petrolatum is used, and in another, a liquid soap is used, such as LIQUID IVORY® soap from Proctor & Gamble, Cincinnati, Ohio.

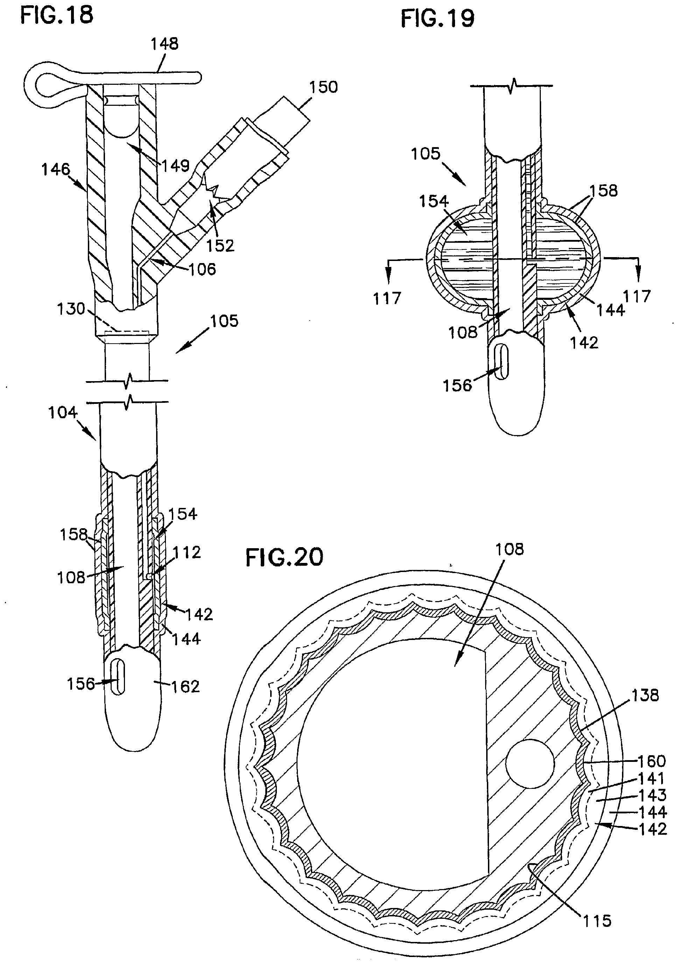

When the intermediate tubes 103 are removed from this first bath of removable bond preventing agent, the agent adheres to the outer surface 114 of the intermediate tube 103, and enters the capillary lumen access opening 112 and runs up into the capillary lumen 106 (as shown in FIG. 13). In one embodiment the agent is petrolatum, which is heated to about 140°-160° F (60°-71° C). In an embodiment, the petrolatum is heated to about 150° F (65.6°C). At these temperatures, the petrolatum will run up into the capillary lumen 106 through the capillary lumen access opening 112 with the assistance of the "capillary effect", which draws the fluid into the capillary lumen 106 to the level of the petrolatum in the first tank. As the intermediate tubes 103 are withdrawn from the hot petrolatum, petrolatum on each tube cools and solidifies to form a semi-solid coating 138 on the outer surface 114 and a semi-solid filling 134 in the capillary lumen 106 and the capillary lumen access opening 112 which cooperate to plug the capillary lumen access opening 112. In an alternate embodiment, the bond preventing agent in the first tank is liquid soap at room temperature (about 62°-74°F / 16.7°- 23.3°C). When the tubes 103 are withdrawn from the first dip tank, the liquid soap forms of semi-solid just as the hot petrolatum did as it cooled. Although both of these bond preventing agents are effective, there is some advantage to using the soap because it does not require the added expense for heating. Furthermore, in certain embodiments, soap is easier to remove from the capillary lumen 106 and the balloon cavity 154 (as shown in FIG. 18).

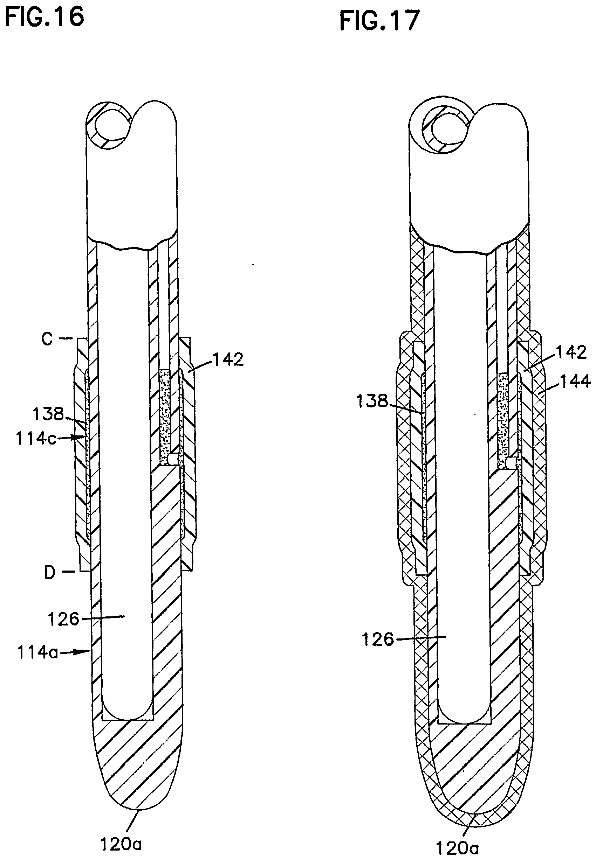

After the intermediate tubes 103 are coated and the capillary lumen access openings 112 are plugged with bond preventing agent in this manner, the tubes 103 are then dipped in a series of dip tanks provided to remove the bond preventing agent from a portion 114a of the outer surface 114 below the line designated B. After this portion 114a of the outer surface 114 is substantially stripped of any residue of the bond preventing agent, the intermediate tubes 103, now partially coated with bond preventing agent between the lines designated A and B as shown in FIG. 15, are dipped in a polymeric bonding composition, such as silicone rubber, in a step or steps provided to coat the intermediate tube 103. The catheter is dipped so that the silicone rubber covers up to line C as shown in FIG. 16. In some embodiments this can be about 0.25 inches above the top of the band 138 of bond preventing agent. Then a solvent is used to remove deposited balloon compound below line D of FIG. 16. Suitable solvents can include xylene or toluene. This deposition process can be repeated until the balloon area (balloon layer) is the desired diameter relative to the shaft of the catheter. In an embodiment the difference in diameters is less than or equal to about 4 French sizes (e.g., about 0.052 inch), for example, no more than 4 French sizes (0.052 inch / 1.33 mm).

After a desired amount of silicone rubber is deposited in the balloon layer, the silicone rubber can be cured before further processing steps. Accordingly, in an embodiment, the silicone rubber including the balloon layer is cured before any further layers of silicone rubber are applied to the catheter. However, in other embodiments, curing can be delayed until later points in the processing steps.

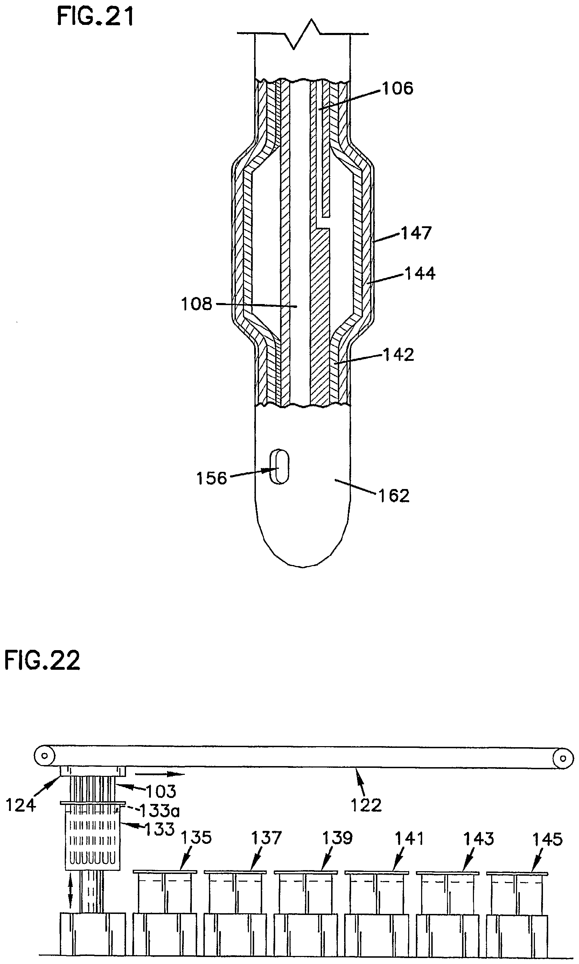

Then the whole length of the intermediate catheter is dipped into a solution of silicone rubber (such as Dow Corning C6-515 or another appropriate balloon compound) creating a second or sheath layer 144 (also known as a second overcoat layer). By applying silicone rubber to the entire length of the shaft, the balloon is thickened but the difference in thickness between the balloon and the shaft is maintained. Optionally, the catheter is then dipped into a solution of thin finish-type silicone rubber (such as Dow Corning 4720) so that the finish-type silicone rubber covers the entire length of the catheter shaft and creates a finish layer 147 (as shown in FIG. 21). This finish layer provides beneficial tactile properties to the exterior of the catheter.

In subsequent steps, the proximal end 130 of the balloon catheter shaft 104 is secured to an end piece 146 to form a completed Foley catheter 105 (shown in FIG. 18). The end piece 146 can include a cap 148 for closing a proximal end access opening 149 to the fluid conduit lumen 108 and can be equipped with a luer valve 150 for engagement in and closure of the proximal capillary lumen access upper opening 152 communicating with the capillary lumen 106. Prior to the attachment of the end piece 146 to the balloon catheter 104 to form the completed Foley catheter 105, the completed balloon catheter 104 is typically allowed to air dry to permit solvents in the first layer 142 (or balloon layer or first overcoat layer) and the second layer 144 (or sheath layer or second overcoat layer) to evaporate and is subsequently cured at an elevated temperature. Care is taken to keep the curing temperature below the boiling temperatures of the solvent so as to prevent unsightly bubbling of the solvent within the balloon layer 142 and the sheath layer 144. The completed Foley catheter 105 also includes a fluid conduit access opening 156 in an exterior surface 162 of the completed Foley catheter 105. The fluid conduit access opening 156 communicates with the fluid conduit lumen 108.

In an embodiment of the present invention, the end piece 146 is made by a process of injection molding. Typically, the proximal end 130 of the balloon catheter shaft 104 is inserted into the injection molding apparatus after the sheath layer 144 has been cured. The polymeric bonding composition, such as silicone rubber, is then injected into the mold (not shown) and the end piece 146 is molded onto the proximal end 130 of the balloon catheter shaft 104 to make the completed Foley catheter 105 shown in FIG. 18.

The balloon portion 158 of the balloon layer 142 and the sheath layer 144 is the portion that is not bonded to the outer surface 114 of the intermediate tube 103. The balloon portion 158 of the balloon layer 142 and the sheath layer 144 cooperates with the portion 114c of the outer surface 114 which remained coated with the bond preventing agent prior to the step of dipping the intermediate tube 103 in the polymeric bonding composition, to define a balloon cavity 154. The balloon cavity 154 communicates with the capillary lumen 106 via the capillary lumen access opening 112. When a fluid is pumped or injected into the capillary access lumen 106, the balloon portion 158 and the balloon cavity 154 are expanded. The bond preventing agent can be removed from the balloon lumen 154 and the capillary lumen 106 by using a hot aqueous solution.

Any of variety of known tests can be used to ensure that there are no leaks in the balloon portion 158 of the finished catheters 105. After testing is completed, the catheters 105 that have passed all tests, are then packaged, such as in a material which breathes such as Tyvek® (from DuPont), and boxed. The boxes can then be sterilized with ETO (Ethylene Oxide) and then stored for shipment.

In an embodiment of the present invention, ribs are formed in the balloon portion 158 of the catheter. For this embodiment, the extruded double lumen tube 102 used to make the intermediate tube 103 is a tube which has a series of generally parallel undulations or channels running generally parallel with the longitudinal axis of the tube 103. When such a tube is used, a Foley catheter having ribs 160 on the inner surface on the balloon portion of the completed Foley catheter will result because the bond preventing coating 138 on the intermediate tube will reciprocate the undulations 115 in the ribbed outer surface 114 of the intermediate tube 103. Embodiments of the catheter made with ribs may or may not have the layer of balloon type silicone rubber coating the entire length of the catheter shaft as described above. As the ribs can serve to prevent cuffing, the extra thickness provided by the additional layer of silicone may be unnecessary depending on the application. Therefore, in some embodiments, the catheter includes both the layer of silicone rubber (or sheath layer) covering the entire catheter shaft and the ribs in the balloon layer.

In some embodiments, the ribs are made of a silicone rubber having different properties than the silicone rubber used for the rest of the balloon. For example, the silicone rubber used to make the ribs can be less pliable than the silicone rubber used to make the rest of the balloon. While not intending to be bound by theory, it is thought that by creating such ribs in the balloon in a direction parallel to the catheter shaft that the stretching of the balloon in that direction is limited resulting in a balloon that resisting cuffing. Referring now to FIG. 20, when making the balloon layer 142, a less pliable silicone rubber is used directly over the bond preventing coating 138, so that an inner region 141 which includes the ribs 160 is first formed, then the rest of the inner layer is formed with regular balloon compound so that the balloon layer 142 also includes a second region 143 of silicone rubber.

In the Applicants' use of the methods of the present invention, balloon fabrication can be almost completely automated. Entire sets of balloon catheters 104 are manufactured simultaneously. The pallet 124 has 400 spring steel support rods 126 attached to a pallet in 20 rows of 20 rods, wherein each of the rods 126 is about 1 inch from each adjacent rod. Double lumen tubing (not shown) can be made by an extrusion process which is known to those of skill in the art. The tubes 102 are cut to length as the tubing leaves the extruder (not shown). An opening 112 is created in the outer surface 114, such as with a hollow drill bit or tube (not shown), so as to communicate with the capillary lumen 106. The distal portion 106a of the capillary lumen 106, located between the distal end 116 of the tube 102 and the capillary lumen access opening 112, is injected with a measured amount of a polymeric bonding composition, such as silicone rubber, so that the distal portion 106a is filled and sealed. A rounded tip 120 can be formed at the distal end 116 of the double lumen tube 102 by inserting the tube 102 in a molding device (not shown).

In some embodiments of the present method, 400 of the intermediate tubes 103 are then mounted vertically on rigid spring steel support rods 126 on a pallet 124 in the manner previously described. The pallet 124 is then moved via a transporting mechanism 122 (see FIG. 22) over a series of dip tanks as follows in one of these embodiments:
(A) The pallet 124 is stopped over a first tank 133, which contains white USP petrolatum heated to about 67° C (about 150° F). The tank is raised so as to immerse the intermediate tubes 103 into the petrolatum to such a depth that the petrolatum reaches the proximal end of the desired balloon location. The dip tank 133 is then lowered and a portion of the outer surface 114 of the intermediate tubes 103 are coated with petrolatum. This portion extends from the point at which the proximal end of the balloon portion 158 will begin to the distal end of the tip 120 of the intermediate tube 103. An intermediate tube after this step is as shown in FIG. 13.
(B) The pallet 124 is then automatically advanced and stopped over a second dip tank 135 which contains white USP petrolatum heated to about 120° C (about 250° F). The second dip tank 135 is raised so as to immerse the intermediate tubes 103 into the super-heated petrolatum so that the super-heated petrolatum comes into contact with the petrolatum coating on outer surface 114 of the intermediate tube 103 from the prior dipping step up to a location where a distal end of the balloon portion 158 will end. The second dip tank 135 is then lowered. This dipping step causes the coating of petrolatum from the prior dipping step to be largely removed from a portion 114a of the outer surface 114 of the intermediate tube 103 from a location where the distal end of the balloon lumen 154 will be located (designated by line B) to the distal end 120a of the tip 120 of the intermediate tube 103. Some residual petrolatum may remain on the outer surface 114 of the intermediate tube 103 in this portion 114a of the outer surface 114. However, most of the petrolatum is removed.
(C) The pallet 124 is then automatically advanced and stopped over a third dip tank 137 containing mineral spirits heated to about 200° F. The third dip tank 137 is then raised so as to immerse the intermediate tubes 103 into the mineral spirits to the same depth as they were immersed in the super-heated petrolatum in the second dip tank 135. The tank 137 is then lowered and all but a trace amount of the petrolatum is removed from the portion 114a of the outer surface 114 below the portion 114c of the outer surface 114, which will eventually be proximate the balloon lumen 154.
(D) The pallet 124 is then automatically advanced and stopped over a fourth dip tank 139 containing a volatile organic solvent such as toluene, trichloroethane or the like. The fourth tank 139 is then raised to immerse the intermediate catheters 103 to the same depth as previously immersed in the second and third tanks 135 and 137, thereby removing essentially all traces of the petrolatum from this portion 114a of the outer surface 114. The intermediate catheter tube 103 now has a band 138 of semi-solid petrolatum located around the axial circumference of the intermediate tube 103 in the location where the balloon cavity 154 will be created. The petrolatum not only coats the portion 114c of the outer surface 114 located in this area, but also fills a portion of the capillary lumen 106 and plugs the capillary lumen access opening 112, which will eventually be used to inflate the balloon portion 158 of the completed Foley catheter 105. An intermediate tube after this step is as shown in FIG. 15.
(E) The pallet 124 is then lowered and automatically advanced to a fifth dip tank 141 containing a heptane dispersed solution of silicone rubber (such as Dow Corning C6-515 or another appropriate balloon compound). The fifth tank 141 is then raised so that the balloon compound covers the balloon area. In some embodiments this can be about 0.25 inches above the top of the band 138 of bond preventing agent.
   Optionally, where it is desired to create ribs on the interior of the balloon that are of a less pliable silicone rubber than the rest of the balloon, the first time of performing step (E) is done by using a dip tank filled with a dispersed solution of silicone rubber that is less pliable than the standard balloon compound used. For example, a higher modulus silicone such as a 50/50 mixture of Dow Corning Q7-4850 and Dow Corning Q7-4720 can be used for initial dips. Thereafter, when step (E) is repeated, the normal balloon compound is used. This results in a balloon with ribs wherein the ribs are less pliable than the rest of the balloon.
(F) The pallet 124 is then advanced to a sixth dip tank 143 containing a solvent effective to remove deposited balloon compound. Suitable solvents include xylene or toluene. The sixth tank 143 is then raised so that the solvent removes the balloon compound below the balloon area. In some embodiments, this can be about 0.25 inches below the top of the band 138 of bond preventing agent. At this point the pallet can be air dried to remove solvents for approximately 30 minutes. Then steps (E) and (F) can be repeated until the balloon area is the desired diameter relative to the shaft of the catheter. In an embodiment this is less than or equal to about 4 French sizes (e.g., about 1.33 mm / 0.052 inch), for example, no more than 4 French sizes (1.33 mm / 0.052 inch), larger than the diameter of the shaft. The number of times that steps (E) and (F) are repeated depends on the type of silicone used for the balloon, the viscosity of the dip solution used, and other factors. In an embodiment, steps (E) and (F) are performed twice.
   Optionally, after step (F), the silicone rubber that was applied during step (F) can be cured before further processing. Accordingly, in an embodiment of the invention, a step of curing the silicone rubber occurs between steps (F) and (G). One of skill in the art will appreciate that there are many methods of curing silicone rubber. By way of example, the silicone rubber can be cured through a heat cure step for approximately two hours at a temperature just below the boiling point of any solvent used in any of the silicone rubber dip solutions.
(G) The pallet 124 is then advanced to a seventh dip tank 145 containing a heptane dispersed solution of silicone rubber (such as Dow Corning C6-515 or another appropriate balloon compound). The seventh tank 145 is then raised so that the balloon compound covers the entire length of the catheter shaft. Then the balloon compound is allowed to air dry for a period of about 30 minutes. This step can be repeated until the ultimate desired thickness of the balloon is achieved. By applying balloon compound to the entire length of the shaft, the balloon is thickened but the same difference in thickness is maintained between the balloon and the shaft as was established in step (F).
(H) Optionally, the pallet can be advanced to an eighth dip tank (not shown) containing a thin finish-type silicone rubber (such as Dow Corning 4720). The eighth tank would be raised so that the finish-type silicone rubber covers the entire length of the catheter shaft. This layer provides beneficial tactile properties to the exterior of the catheter.
(I) The pallet is then advanced through a drying area where solvents are allowed to evaporate for approximately two hours, and then through a heat cure step for approximately two hours, where the balloon catheters 104 formed by this process are cured at a temperature just below the boiling point of any solvent used in any of the silicone rubber dip solutions. For toluene this temperature is about 93.33°C (200°F) though other temperatures can be used. One of skill in the art will appreciate that the drying and curing times are approximate and can be varied depending on the specific materials and solvents used.
(J) After the heat cure, the balloon catheters 104 are allowed to cool and are then removed from the support rods 126. The proximal ends 130 of each of the balloon catheters 104 is then inserted into an injection molding apparatus (not shown), which forms the end piece 146 of the completed Foley catheter 105.
(K) The completed Foley catheters 105 are then finished by punching a fluid conduit access opening 156 in the exterior surface 162 such that it communicates with the fluid conduit lumen 108 in a location below or distal to the balloon portion 158.
(L) The completed Foley catheters 105 are then sent through a test sequence, during which the balloon portion 158 of each completed Foley catheter 105 is inflated and the petrolatum band 138 within the balloon cavity 154 is largely removed by a hot aqueous solution.

One of skill in the art will appreciate that while the methods are described as they can be practiced in an automated or semi-automated fashion, the methods can also be practiced in a non-automated fashion as well with dipping steps and the like being performed by hand.

The disclosures of USPN 5,670,111 (Conway et al.), USPN 5,360,402 (Conway et al.), USPN 5,269,770 (Conway et al.), USPN 5,261,896 (Conway et al.), USPN 5,137,671 (Conway et al.), and USPN 5,098,379 (Conway et al.).

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the phrase "adapted and configured" describes a system, apparatus, or other structure that is constructed or configured to perform a particular task or adopt a particular configuration to. The phrase "adapted and configured" can be used interchangeably with other similar phrases such as arranged and configured, constructed and arranged, adapted, constructed, manufactured and arranged, and the like.

All publications and patent applications in this specification are indicative of the level of ordinary skill in the art to which this invention pertains.

The invention has been described with reference to various specific embodiments and techniques. However, it should be understood that many variations and modifications may be made while remaining within the scope of the invention.

## Claims

1. A catheter comprising:
a central layer (103) providing a catheter shaft (104) and having a catheter tip (120), an exterior surface (162) and an interior surface and defining a first lumen (108) and a second lumen (106);
a balloon layer (142) surrounding the central layer (103), the balloon layer (142) including a balloon portion (158), the balloon portion (158) being the portion which is not bonded to the exterior surface (162) of the central layer (103);
the balloon portion (158) being in the form of a cavity disposed between the exterior surface (162) of the central layer (103) and the balloon layer (142);
wherein the balloon cavity (154) is in fluid communication with the second lumen (106);
wherein the balloon layer (142) and the central layer (103) are joined together at a distal end (116) and at a proximal end (130) of the balloon cavity (154) relative to the catheter tip (120); and
a sheath layer (144) surrounding the balloon layer (142) and extending at least over the balloon layer (142);
**characterized in that**, the balloon layer (142) is provided on the catheter shaft (104) just in the area (C to D) of the balloon portion (158) and the sheath layer (144) is provided on the catheter shaft (104) over the entire length of the catheter shaft (104) and the balloon layer (142) does not cover the entire length of the catheter shaft (104) to be inserted into the patient.

2. The catheter of claim 1, wherein the central layer (103), the balloon layer (142), and the sheath layer (144) comprise silicone rubber.

3. The catheter of claim 1, wherein the first lumen (108) comprises a fluid passage lumen and the second lumen (106) comprises a balloon inflation lumen,

4. The catheter of claim 1, wherein the balloon layer (142) comprises silicone rubber.

5. The catheter of claim 1, wherein the sheath layer (144) comprises silicone rubber.

6. The catheter of claim 1, wherein the sheath layer (144) extends over the entire portion of the catheter to be inserted into the patient.

7. The catheter of claim 1, further comprising a finish layer (147) disposed on the sheath layer (144).

8. The catheter of claim 7, wherein the finish layer (147) comprises silicone rubber.

9. The catheter of claim 1, wherein the difference between the diameter of the central layer (103) and the balloon layer (142) is less than or equal to 0.052 inches.

10. A method for forming a cuff-resistant catheter comprising:
providing a silicone rubber tube with a first lumen (108) and a second lumen (106);
cutting the silicone rubber tube it to a desired length, wherein the cut silicone rubber tube has a distal end (116) and a proximal end (130);
creating an access opening between an outer surface of the double lumen tube and the first lumen (108);
filling the first lumen (108) with a polymeric bonding composition between the access opening and the distal end (116) of the silicone rubber tube;
providing a catheter tip (120) on the distal end (116) of the silicone rubber tube;
applying a bond preventing agent to the silicone rubber tube in a desired area;
applying a balloon coat of silicone rubber to the silicone rubber tube In an area overlapping the bond preventing agent but not over the entire length of the silicone rubber tube;
using a solvent to remove deposited balloon coat from a portion of the length of the silicone rubber tube proximal the end of the silicone rubber tube;
applying a sheath coat (144) of silicone rubber over the entire length of the silicone rubber tube;and
providing an end piece onto the proximal end (130) of the silicone rubber tube.

11. The method of claim 10, further comprising the step of applying a finish coat (147) of silicone rubber over the entire length of the silicone rubber tube wherein the finish coat comprises a silicone rubber that is different from the silicone rubber of the sheath coat (144).

12. The method of claim 10 wherein the steps of applying bond preventing agent, applying a balloon coat of silicone rubber, and applying a sheath coat (144) of silicone rubber are automated.

## Patentansprüche

1. Katheter umfassend:
eine mittlere Schicht (103), die einen Katheterschaft (104) bereitstellt und eine Katheterspitze (120), eine Außenfläche (162) und eine Innenfläche aufweist und ein erstes Lumen (108) und ein zweites Lumen (106) definiert;
eine Ballonschicht (142), die die mittlere Schicht (103) umgibt, wobei die Ballonschicht (142) einen Ballonabschnitt (158) enthält, wobei der Ballonabschnitt (158) der Abschnitt ist, der nicht an die Außenfläche (162) der mittleren Schicht (103) gebunden ist;
wobei der Ballonabschnitt (158) in Form eines Hohlraumes vorliegt, der zwischen der Außenfläche (162) der mittleren Schicht (103) und der Ballonschicht (142) angeordnet ist;
wobei der Ballonhohlraum (154) sich in Fluidkommunikation mit dem zweiten Lumen (106) befindet;
wobei die Ballonschicht (142) und die mittlere Schicht (103) an einem distalen Ende (116) und an einem proximalen Ende (130) des Ballonhohlraums (154) im Verhältnis zu der Katheterspitze (120) zusammengefügt sind; und
eine Mantelschicht (144), die die Ballonschicht (142) umgibt und sich mindestens über die Ballonschicht (142) erstreckt;
**dadurch gekennzeichnet, dass** die Ballonschicht (142) auf dem Katheterschaft (104) nur in dem Bereich (C bis D) des Ballonabschnitts (158) bereitgestellt wird und die Mantelschicht (144) auf dem Katheterschaft (104) über die gesamte Länge des Katheterschafts (104) bereitgestellt wird und die Ballonschicht (142) die gesamte Länge des Katheterschaft (104), die in den Patienten eingeführt werden soll, nicht bedeckt.

2. Katheter nach Anspruch 1, wobei die mittlere Schicht (103), die Ballonschicht (142) und die Mantelschicht (144) Siliconkautschuk umfassen.

3. Katheter nach Anspruch 1, wobei das erste Lumen (108) ein Fluiddurchgangslumen umfasst und das zweite Lumen (106) ein Ballonaufblählumen umfasst.

4. Katheter nach Anspruch 1, wobei die Ballonschicht (142) Siliconkautschuk umfasst.

5. Katheter nach Anspruch 1, wobei die Mantelschicht (144) Siliconkautschuk umfasst.

6. Katheter nach Anspruch 1, wobei die Mantelschicht (144) sich über den gesamten Abschnitt des Katheters, der in den Patienten eingeführt werden soll, erstreckt.

7. Katheter nach Anspruch 1, der ferner eine Abschlussschicht (147) umfasst, die auf der Mantelschicht (144) angeordnet ist.

8. Katheter nach Anspruch 7, wobei die Abschlussschicht (147) Siliconkautschuk umfasst.

9. Katheter nach Anspruch 1, wobei der Unterschied zwischen dem Durchmesser der mittleren Schicht (103) und der Ballonschicht (142) weniger als oder gleich 0,052 Zoll ist.

10. Verfahren zum Bilden eines gegen Manschettenbildung resistenten Katheters, umfassend:
Bereitstellen einer Silikonkautschukröhre mit einem ersten Lumen (108) und einem zweiten Lumen (106); Schneiden der Silikonkautschukröhre auf eine erwünschte Länge, wobei die geschnittene Silikonkautschukröhre ein distales Ende (116) und ein proximales Ende (130) aufweist;
Erzeugen einer Zugangsöffnung zwischen einer äußeren Oberfläche der doppelten Lumenröhre und dem ersten Lumen (108);
Füllen des ersten Lumens (108) mit einer polymeren Bindungszusammensetzung zwischen der Zugangsöffnung und dem distalen Ende (116) der Siliconkautschukröhre;
Bereitstellen einer Katheterspitze (120) an dem distalen Ende (116) der Siliconkautschukröhre;
Aufbringen eines bindungsverhindernden Mittels auf die Siliconkautschukröhre in einem erwünschten Bereich;
Aufbringen einer Ballonbeschichtung aus Siliconkautschuk auf die Siliconkautschukröhre in e4inem Bereich, der das bindungsverhindernde Mittel überlappt, jedoch nicht die gesamte Länge der Siliconkautschukröhre bedeckt;
Verwenden eines Lösungsmittels zum Entfernen von aufgebrachter Ballonbeschichtung von einem Abschnitt der Länge der Siliconkautschukröhre proximal zum Ende der Siliconkautschukröhre;
Aufbringen einer Mantelbeschichtung (144) aus Siliconkautschuk über die gesamte Länge der Siliconkautschukröhre; und
Bereitstellen eines Endstücks auf dem proximalen Ende (130) der Siliconkautschukröhre.

11. Verfahren nach Anspruch 10, ferner den Schritt umfassend des Aufbringens einer Abschlussbeschichtung (147) aus Siliconkautschuk über die gesamte Länge der Siliconkautschukröhre, wobei die Abschlussbeschichtung einen Siliconkautschuk umfasst, der von dem Siliconkautschuk der Mantelbeschichtung (144) verschieden ist.

12. Verfahren nach Anspruch 10, wobei die Schritte des Aufbringens des bindungverhindernden Mittels, Aufbringens einer Ballonbeschichtung aus Siliconkautschuk und Aufbringens einer Mantelbeschichtung (144) aus Siliconkautschuk automatisiert sind.

## Revendications

1. Sonde comprenant :
une couche centrale (103) pourvoyant à une gaine (104) de sonde et comportant un bout (120) de sonde, une surface extérieure (162) et une surface intérieure et délimitant une première lumière (108) et une seconde lumière (106) ;
une couche (142) de ballonnet entourant la couche centrale (103), la couche (142) de ballonnet comprenant une partie (158) de ballonnet, la partie (158) de ballonnet étant la partie qui n'est pas collée à la surface extérieure (162) de la couche centrale (103) ;
la partie (158) de ballonnet ayant la forme d'une cavité disposée entre la surface extérieure (162) de la couche centrale (103) et la couche (142) de ballonnet, dans lequel la cavité (154) de ballonnet est en communication fluidique avec la seconde lumière (106), dans lequel la couche (142) de ballonnet et la couche centrale (103) sont assemblées au niveau de l'extrémité distale (116) et de l'extrémité proximale (130) de la cavité (154) de ballonnet par rapport au bout (120) de sonde ; et
une couche (144) de gaine entourant la couche (142) de ballonnet et s'étendant au moins par-dessus la couche (142) de ballonnet,
**caractérisé en ce que** la couche (142) de ballonnet est disposée sur la gaine (104) de sonde juste dans la zone (C à D) de la partie (158) de ballonnet et la couche (144) de gaine est disposée sur la gaine (104) de sonde sur toute la longueur de la gaine (104) de sonde et la couche (142) de ballonnet ne couvre pas toute la longueur de la gaine (104) de sonde à introduire dans le patient.

2. Sonde selon la revendication 1, dans lequel la couche centrale (103), la couche (142) de ballonnet et la couche (144) de gaine sont constituées de caoutchouc de silicone.

3. Sonde selon la revendication 1, dans lequel la première lumière (108) comprend une lumière de passage de fluide et la seconde lumière (106) comprend une lumière de gonflage de ballonnet.

4. Sonde selon la revendication 1, dans lequel la couche (142) de ballonnet est constituée de caoutchouc de silicone.

5. Sonde selon la revendication 1, dans lequel la couche (144) de gaine est constituée de caoutchouc de silicone.

6. Sonde selon la revendication 1, dans lequel la couche (144) de gaine s'étend sur toute la partie du cathéter à introduire dans le patient.

7. Sonde selon la revendication 1, comprenant en outre une couche (147) de finissage entourant la couche (144) de gaine.

8. Sonde selon la revendication 7, dans lequel la couche (147) de finissage est constituée de caoutchouc de silicone.

9. Sonde selon la revendication 1, dans lequel la différence entre le diamètre de la couche centrale (103) et la couche (142) de ballonnet est inférieure ou égale à 1,3208 mm.

10. Procédé de formation d'une sonde résistant à l'engorgement, comprenant les opérations consistant à :
produire un tube en caoutchouc de silicone ayant une première lumière (108) et une seconde lumière (106) ;
couper le tube en caoutchouc de silicone à la longueur souhaitée, dans lequel le tube en caoutchouc de silicone coupé comporte une extrémité distale (116) et une extrémité proximale (130) ;
créer une ouverture d'accès entre la surface extérieure du tube à double lumière et la première lumière (108) ;
remplir la première lumière (108) avec une composition de collage polymère entre l'ouverture d'accès et l'extrémité distale (116) du tube en caoutchouc de silicone ;
disposer un bout (120) de sonde sur l'extrémité distale (116) du tube en caoutchouc de silicone ;
appliquer au le tube en caoutchouc de silicone, dans une zone souhaitée, un agent empêchant de coller ;
appliquer au tube en caoutchouc de silicone un enduit de ballonnet en caoutchouc de silicone dans une zone chevauchant l'agent empêchant de coller mais pas sur toute la longueur du tube en caoutchouc de silicone ;
utiliser un solvant pour enlever l'enduit de ballonnet déposé d'une partie de la longueur du tube en caoutchouc de silicone à proximité de l'extrémité du tube en caoutchouc de silicone ;
appliquer une couche (144) de gaine en caoutchouc de silicone sur toute la longueur du tube de caoutchouc de silicone ; et
disposer une pièce terminale sur l'extrémité distale (130) du tube en caoutchouc de silicone.

11. Procédé selon la revendication 10, comprenant en outre l'étape consistant à appliquer une couche (147) de finissage en caoutchouc de silicone sur toute la longueur du tube de caoutchouc de silicone, dans lequel la couche de finissage est constituée d'un caoutchouc de silicone qui est différent du caoutchouc de silicone de la couche (144) de gaine.

12. Procédé selon la revendication 10, dans lequel les étapes d'application de l'agent empêchant de coller, d'application de l'enduit de ballonnet en caoutchouc de silicone et d'application de la couche (144) de gaine en caoutchouc de silicone sont automatisées.
